# EUROPÄISCHE PATENTSCHRIFT

(11) **EP 1 786 929 B1**
(45) Veröffentlichungstag und Bekanntmachung des Hinweises auf die Patenterteilung: **22.06.2016**
(21) Anmeldenummer: 05787279.8
(22) Anmeldetag: 17.08.2005
(51) Int. Cl.: C12Q 1/68

(54) **VERFAHREN ZUR DIAGNOSE VON EIERSTOCKKREBS**
METHOD FOR THE DIAGNOSIS OF A OVARIAN CANCER
PROCEDE POUR LE DIAGNOSTIC D'UN CANCER DE L'OVAIRE

(30) Priorität: 19.08.2004 AT 13982004
(43) Veröffentlichungstag der Anmeldung: 23.05.2007
(73) Patentinhaber: ZEILLINGER, Robert, 2602 Blumau-Neurisshof (AT)
(72) Erfinder: ZEILLINGER, Robert, 2602 Blumau-Neurisshof (AT); FABJANI, Gerhild, 1100 Wien (AT)
(74) Vertreter: Redl, Gerda
(86) Internationale Anmeldenummer: PCT/EP2005/008908
(87) Internationale Veröffentlichungsnummer: WO 2006/018290

(56) Entgegenhaltungen:
- WO-A-01/75177
- US-A1- 2003 003 479
- STIMPFL M ET AL.: "Expression of mucins and cytokeratins in ovarian cancer cell lines" CANCER LETTERS, Bd. 145, 1999, Seiten 133-141, XP002360224 in der Anmeldung erwähnt
- RAYNOR M ET AL.: "Optimisation of the RT-PCR detection of immunomagnetically enriched carcinoma cells" BMC CANCER, Bd. 2, 2002, Seiten 14-22, XP002360225
- TSUCHIYA A ET AL.: "Expression profiling in ovarian clear cell carcinoma", AMERICAN JOURNAL OF PATHOLOGY, vol. 163, no. 6, December 2003 (2003-12), pages 2503-2512,
- KUAN C-T ET AL.: "EGF mutant receptor vIII as a molecular target in cancer therapy", ENDOCRINE-RELATED CANCER, vol. 8, 2001, pages 83-96,
- KLEIN C A: "The systemic progression of human cancer: A focus on the individual disseminated cancer cell - the unit of selection", ADVANCES IN CANCER RESEARCH, vol. 89, 2003, pages 35-67,

## Beschreibung

Die Erfindung betrifft ein Verfahren zur Diagnose einer Krebserkrankung, insbesondere mit Eierstockkrebs als Primärtumor, durch den Nachweis von disseminierten Tumorzellen in einer biologischen Probe eines Patienten, indem untersucht wird, ob eine Genexpression auf RNA- oder Proteinebene für ein oder mehrere bestimmte Gene (Markergene) in dieser biologischen Probe nachweisbar ist.

Weiter wird ein Kit beschrieben zur Diagnose einer Krebserkrankung bei einem Patienten, insbesondere mit Eierstockkrebs als Primärtumor, oder zum Feststellen ob und wie gut ein Patient auf die Behandlung einer Krebserkrankung, insbesondere mit Eierstockkrebs als Primärtumor, anspricht, wobei der Kit Reagenzien zur Bewertung der Genexpression auf RNA- ebene für ein oder mehrere bestimmte Markergene in einer biologischen Probe des Patienten enthält.

Bei Patienten mit soliden Tumoren haben 30% zum Zeitpunkt der Diagnose auffindbare Metastasen. Metastasierung ist die Verteilung oder die Bewegung von Tumorzellen vom Ort des (Primums) Primärtumors in einen anderen Bereich des Körpers. Etwa die Hälfte der Patienten können allein durch Behandlung des Primärtumors geheilt werden. Die Verbleibenden haben zum Zeitpunkt der Diagnose nicht auffindbare Metastasen, die zu einem Fortschreiten der Erkrankung führen können.

Die Metastasierung ist ein vielstufiger Vorgang, bei dem Tumorzellen die Tumorgrenze, das Parenchym und die Basalmembran durchqueren, über Lymph- und Blutgefäße sich im Körper verbreiten, in Fremdgewebe festsetzen, wo sie Metastasen ausbilden und Angiogenese initiieren. Das aktuelle Modell für Metastasierung geht von einem niedrigen Metastasenpotential der meisten Zellen im Primärtumor aus. Nur wenige Tumorzellen sind aufgrund von somatischen Mutationen in der Lage zu metastasieren. Die Fähigkeit von Krebszellen zu wandern, erlaubt ihnen im Gewebe die Position zu ändern, in das Lymphund Blutgefässsystem einzutreten, um sich im Körper zu verbreiten und Metastasen in entfernten Organen zu bilden. Tumorzellen, die in das Lymphsystem abwandern, setzen sich oft in der nächsten Lymphknotengruppe fest, wo sie wachsen bevor sie sich weiter in entferntere Körperregionen verteilen. Man nimmt an, dass weniger als 1 von 10000 Zellen, die aus dem Primärtumor auswandern überleben, aber dies genügt um Sekundärtumore irgendwo Im Körper zu bilden. Tumorzellen die in den Blutkreislauf abwandern, müssen überleben bis sie sich an die Mikrogefäßwände des Sekundärortes heften. Von dort können sie in das umliegende Gewebe einwandern, wo sie zu Mikrometastasen heranwachsen. Die Ausbildung neuer Blutgefässe ist eine Vorbedingung für die Entwicklung von makroskopisch sichtbaren Tumoren. Von dieser Metastase können Tumorzellen wiederum in das Kreislaufsystem eintreten. Entsprechend dieser Theorie, weist das Vorhandensein von disseminierten Tumorzellen im Blut, ob sie vom Primärtumor stammen oder von einer Metastase, auf eine bestehende Krebserkrankung hin.

Die Behandlung von Krebs inkludiert die Behandlung von Metastasen, die nicht durch Operation allein geheilt werden können. Diese adjuvante Therapie zielt auf die Eliminierung von disseminierten Tumorzellen (DTC) und entfernten Metastasen. DTC können in Körperflüssigkeiten und Geweben wie Blut, Knochenmark, Lymphknoten, oder der Bauchhöhle von Krebspatienten selbst nach Operation mit kurativem Charakter festgestellt werden und dieser Umstand wird als "Minimalrestkrankheit" (minimal residual disease) beschrieben. Die Eliminierung von DTC wird eine wesentliche Herausforderung für zukünftige Therapien gegen Krebs sein, um die Bildung von Metastasen zu verhindern. Das Auffinden von DTC spiegelt die biologische Aktivität der Tumorerkrankung wider und kann auch helfen, die Wirksamkeit verschiedener adjuvanter Therapien vorherzusagen. Die überwiegende Mehrheit der Tumorzellen, die in Blutgefässe eintreten, wird durch eine Vielzahl von Mechanismen getötet. Es ist nur wenig über die Dynamik der Dissemination von Tumorzellen in den Blutstrom bekannt: Ob es ein kontinuierliches oder ein diskontinuierliches Ereignis ist, wie viele Zellen In den Blutstrom eintreten und wie viele Zellen die Blutgefässe verlassen. Die prognostische Relevanz der Auffindung von Tumorzellen in Blut ist noch umstritten, obwohl ihr Vorhandensein stark mit einem zunehmenden Risiko für Metastasen für alle Haupttypen von Krebs verbunden ist Die Biologie von DTC blieb weitgehend unerforscht. Dieser Wissensmangel hemmt die Schaffung von wirksamen adjuvanten Therapien für die Beseitigung von DTC.

Da sich die Hinweise häufen, dass die Abwanderung der Tumorzellen in das Blut schon frühzeitig in der Tumorentwicklung stattfindet, ist die Auffindung von DTC In Blut eine der größten Herausforderungen in Bezug auf die Früherkennung von Krebs, insbesondere von Krebsarten, die aufgrund fehlender Frühzeichen der Erkrankung erst in einem sehr weit fortgeschrittenem Stadium diagnostiziert werden, wie z.B. Ovarialkarzinome. Auch für die Auswahl einer optimalen Therapie und das Überwachen der Wirksamkeit einer Behandlung kann das Auffinden von DTC von großer Bedeutung sein. Die Entwicklung eines robusten Tests zur Auffindung von DTC erfordert eine hoch sensitive Screening-Technik, die eine Reihe von informativen Markern analysieren kann. Große Anforderungen werden an diese Marker hinsichtlich ihrer Sensitivität und Spezifität gestellt. Es ist deshalb eine sehr hohe Sensitivität erforderlich, da DTC nur mit einer extrem niedrigen Frequenz von 1 zu 10⁵ - 10⁷ normalen Blutzellen gefunden werden. Daher sollten Markergene verwendet werden, die nicht in normalen Blutzellen exprimiert werden. In den letzten Jahren wurden hoch sensitive Verfahren, wie real time RT-PCR, für den Nachweis von sehr wenigen Transkripten etabliert. Eines der Hauptprobleme bei der Verwendung solcher sensitiven Verfahren für die DTC Auffindung in Blut ist, dass Expressionssignale von normalen Blutzellen auch erhalten werden und so zu falsch positiven Ergebnissen führen. Daraus ergibt sich die Notwendigkeit einer Tumorzellenanreicherung oder sogar -isolation, um das Problem des Hintergrundsignals von normalen Blutzellen zu lösen. Dieses Hintergrundsignal führt zur Überlagerung und damit zum Verschwinden des von den Tumorzellen aufgebauten Signals in der großen Menge normaler Blutzellen Ein anderes evidentes Problem ist, dass den derzeit verfügbaren Tumormarker die Spezifität für bestimmte Krebsarten fehlt. Momentan werden epithelzellspezfische Marker, wie bestimmte Zytokeratine oder das epitheliale Zelladhäsionsmolekül (EpCAM) für die Auffindung von Tumorzellen verwendet, die aber einerseits auch In den nicht epithelialen Blutzellen vorhanden sein können, andererseits in Tumorzellen fehlen können.

Schon 1869 hat Ashworth einen Fall eines verstorbenen Tumorpatienten beschrieben, bei welchem Zellen ähnlichen jenen im Tumor im Blut gefunden wurden. Seit diesem Zeitpunkt ist die Auffindung von disseminierten Tumorzellen von großem Interesse. Beweise häufen sich, dass bereits aus einem Primärtumor eines sehr frühen Stadiums neoplastische Zellen in den Kreislauf abzuwandern. Diese Tumorzellen weisen, abhängig von ihrer Herkunft, spezifische Genexpressionsprofile auf, die sich von jenen normaler Blutzellen unterscheiden. Es sollte daher möglich sein Tumorzellen in peripherem Blut in einem frühen Stadium der Tumorentwicklung zu finden.

Die meisten der verwendeten Techniken sind entweder proteinbasierende Tests, wie Immunozytochemie, oder mRNA-basierende Verfahren, wie RT-PCR (reverse transcription - polymerase chain reaction). Im Vergleich mit proteinbasierenden Tests, haben PCR-Verfahren den großen Vorteil sehr hoher Sensitivität. Eine Vielzahl von PCR-basierenden Techniken wurde für das Auffinden von disseminierten Tumorzellen entwickelt Die Hauptstrategien für das Auffinden von verborgenen Tumorzellen durch PCR ist die Charakterisierung von gewebespezifischen oder tumorspezifischen mRNAs. Verschiedene Zytokeratine (Zytokeratin 19, Zytokeratin 20), epitheliale Membranantigene, wie Mucin 1 (MUC1), das epitheliale Zelladhäsionsmolekül (EpCAM), sowie Tumormarker, wie Carcinoembryonic Antigen (CEA), Her2/neu, Epidermal Growth Factor Receptor (EGFR) wurden verwendet, um Tumorzellen in Blut von Brustkrebs-, Eierstockkrebs, Gebärmutterhalskrebs- und Gebärmutterkrebspatientinnen aufzufinden Bei Verwendung dieser Marker wurde über variierende Auffindungsraten für Tumorzellen berichtet Leider haben diese Tests im Allgemeinen ungenügende Sensitivität und/oder Spezifität. Obwohl PCR ein wirkungsvolles Mittel zum Auffinden von mRNA-Molekülen ist, gibt es bei Verwendung dieser Technik immer noch Grenzen beim Auffinden disseminierter Tumorzellen in peripherem Blut. Das Hauptproblem ist die mangelnde Spezifität der Marker. Da Zytokeratine und epitheliale Membranantigene auch in nicht-epithelialen Blutzellen exprimiert werden können kann Blut von gesunden Individuen falsch positive Resultate liefern. Ein weiteres Problem stellt die normalerweise sehr kleine Anzahl zirkulierender Tumorzellen in einer großen Zahl von Blutzellen dar Es können falsch negative Resultate bei dem hohen Blutzellenhintergrund erhalten werden. Es ist daher von besonderer Bedeutung, die zirkulierenden Tumorzellen zum Nachweis und zur weiteren Charakterisleren anzureichern. Derzeit werden hauptsächlich immun-magnetische Verfahren, Dichtegradientzentrifugationsverfahren und Filtrationsverfahren für die Tumorzellenanrelcherung verwendet. Tests basierend auf einzelnen Markern zum Nachweis zirkulierender Tumorzellen haben im Allgemeinen eine niedrige Auffindungsrate. Das beruht hauptsächlich auf der genetischen Heterogenität der meisten Krebsarten. So gibt es höchstwahrscheinlich keinen einzigartigen Krebsmarker, selbst nicht für eine bestimmte Krebsart. Es werden daher komplexe Tests zur gleichzeitigen Analyse mehrerer Marker benötigt.

Eines der effektivsten Mittel zur Reduktion der Krebsmortalität ist die Früherkennung, die die Behandlungsmögiichkeiten und die Prognose stark verbessert. Ferner besteht eine starke Notwendigkeit, die Behandlungseffizienz bei Krebspatienten zu überwachen. Dies kann durch einen Bluttest erfolgen, der sowonl Früherkennung, als auch information über das Ansprechen auf die Therapie ermöglicht. Blut ist ein ideales Untersuchungsmaterial für die Analytik, da Blutproben leicht gewonnen werden können, und der Nachweis zirkulierender Tumorzellen, im Blut sich sowohl für die Frühdiagnostik, als auch zur Analyse von Verlaufsparametern ideal eignet. Um einen Test für das Auffinden von disseminierten Tumorzellen in Blut zu entwickeln, ist eine hoch sensitive Screeningtechnik mit hoher Probendurchsatzmöglichkelt erforderlich, um informative Marker, die Tumorzellen von Blutzeilen unterscheiden sollen, zu identifizieren.

Obgleich im Stand der Technik, beispielsweise in US 2003/003479 und WO01/75177, Markergene identifiziert und deren Expressionsmuster im Zusammenhang mit Primäreierstockkrebs untersucht werden, wird in diesen Testverfahren kein Zusammenhang zu disseminierten Tumorzellen gelegt. Auch Tsuchiya et al., Am. J.Pathol., vol. 163, pp. 2503 - 2512, 2003, beschreiben ein gleichartiges Verfahren ohne zuvorige Anreicherung disseminierter Tumorzellen und weiterführender Genexpressionsanalyse.

Aufgabe der vorliegenden Erfindung ist es, solche zuverlässigen, genauen und spezifischen Tumormarker für das Auffinden von Tumorzellen in Krebspatienten, insbesondere Patientinnen mit Eierstockkrebs zu finden, um sie in einem Verfahren zur Diagnose von Krebs, insbesondere Elerstockkrebs und zur Überwachung des Ansprechens eines Patienten bei der Behandlung von Krebs, insbesondere Eierstockkrebs, einzusetzen.

Die Aufgabe wird gelöst, indem bei den eingangsgenannten Verfahren und für den eingangsgenannten Kit erfindungsgemäß das bzw. die Markergene aus einer Gruppe von Genen ausgewählt ist bzw. sind, die NR2F2, LEMD1, CALB1, LAMC2, FN1, STMY2, COL4A2, GPRC5A, NTS, GHR, EDNRA, TUSC3, PLEKHC1, RBPMS die EGFR Variante mit der Genbankzugangsnummer NM_005228 und die EGFR-Variante mit der Genbankzugangsnummer K03193.1 enthält.

Von den möglichen Markergenen für FN1 werden in einer bevorzugten Ausführungsform des erfindungsgernäßen Verfahrens und des erfindungsgemäßen Kits die Varianten fibronectin 1 mit der Genbankzugangsnummer XO2761, fibronectin 1 isoform 3 mit der Genbankzugangsnummer NM_002026, und/oder fibronectin 1 isoform 6 mit der Genbankzugangsnummer NM_212474 verwendet.

Das erfindungsgemässe Verfahren kann zur Früherkennung von Eierstockkrebs eingesetzt werden.

Für die genannten Gene findet eine deutliche Expression in Blutproben von Krebspatienten, insbesondere Eierstockkrebspatientinnen, statt, wohingegen diese Gene in Blutproben von gesunden Personen nicht oder nur minimal exprimiert werden. Die Kombination aus mehreren Markergenen bei den Verfahren und im Kit erhöht die Aussagekraft der Verfahren.

Die Gene sind in der folgenden Tabelle näher beschrieben. Spezifisch angeführte Genvarianten sind als solche im Feld "Genmarker" genannt. Andere bekannte Genvarianten, die über das Nachweisverfahren für das betreffende Gen indirekt bestimmt werden, ohne aber zwischen diesen Varianten zu diskriminieren, sind mit der "GenBank Zugangsnummer" angeführt, ohne diese aber im Feld "Genmarker" zu spezifizieren.

| **Genmarker** | **GenBank-Zugangsnummer** | **Beschreibung** |
|---|---|---|
| NR2F2 | NM_021005 | nuclear receptor subfamily 2, group F, member2 |
| PAX8 | BC001060 | paired box gene 8 |
| | NM_003466 | variant 1 "PAX8A" |
| | NM_013951 | variant 2 "PAX8B" |
| | NM_013952 | variant 3 "PAX8C" |
| | NM_013953 | variant 4 "PAX8D" |
| | NM_013992 | variant 5 "PAX8E" |
| LAMC2 | | laminin, gamma 2 |
| | NM_005562 | variant 1 "laminin, gamma 2 isoform a precursor" |
| | NM_018891 | variant 2 "laminin, gamma 2 isoform b precursor" |
| Latrophilin | NM_012302 | latrophilin 2 |
| FN1 | X02761 | fibronectin variant 1 "FN1 Standard" |
| FN1v0 | NM_002026 | fibronectin variant 2 "FN1v89" (fibronectin 1 isoform 3 preproprotein) |
| FN1v89 | NM_212474 | fibronectin variant 3 "FN1v0" (fibronectin 1 lsoform 6 preproprotein) |
| CALB1 | NM_004929.2 | calbindin 1 |
| LEMD1 | NM_001001552 | LEM domain containing 1 |
| STMY2 | NM_2425 | matrix metalloproteinase 10 |
| COL3A1 | NM_000090 | collagen, type III, alpha1 |
| COL4A2 | NM_001846 | collagen, type IV, alpha2 |
| NTS | NM_006183 | neurotensin |
| GPRC5A | NM_003979 | G protein-coupled receptor, family C, group 5, member A, retinoic acid induced 3 |
| GHR | NM_000163 | growth hormone receptor |
| EDNRA | NM_001957 | endothelin receptor type A |
| TUSC3 | NM_006765 | tumor suppressor candidate 3 variant 1 "tumor suppressor candidate 3 isoform a" |
| | NM_178234 | variant 2 "tumor suppressor candidate 3 isoform b" |
| PLEKHC1 | NM_006832 | plekstrin homology domain-containing protein, family member 1 |
| RBPMS | NM_001008710 | RNA-binding protein gene with multiple splicing variant 1 "RNA-binding protein with multiple splicing A" |
| | NM_001008711 | variant 2 "RNA-binding protein with multiple splicing B" |
| | NM_001008712 | variant 3 "RNA-binding protein with multiple splicing isoform C" |
| | NM_006867 | variant 4 "RNA-binding protein with multiple splicing A" |
| TM4FS1 | NM_014220 | transmembrane 4 L six family member 1 |
| EGFR | | epidermal growth factor receptor |
| EGFR 5.5 | NM_005228 (EGFR 5.5) | variant 1 "epidermal growth factor receptor isoform a" |
| EGFR 2.6 | K03193.1 (EGFR 2.6) | variant 2 "aberrant (short) epidermal growth factor receptor" |

Zahlreiche Gene können in verschiedenen Varianten exprimiert werden; so z.B. auch Flbronektin (=FN1). Für die vorliegende Erfindung werden jeweils alle Varianten miteinbezogen, speziell die Varianten "FN1Standard", "FN1v0", "FN1v89", "EGFR5,5" und "EGFR2.6"

Bei einer Untersuchung über die Expression von Kandidatengenen in Blut von Eierstockkrebspatientinnen wurde zuerst in 67 Patientenblutproben und von 17 Blutproben von gesunden Frauen eine Tumorzellanreicherung mit der OnkoQuick-Zentrifugationsmethode (Greiner, Deutschland) vorgenommen. Dann wurde RNA unter Verwendung von RNeasy Micro Kit (Quiagen, Deutschland) isoliert, in cDNA umgeschrieben und danach die cDNA mit linearer Amplifikation vervielfältigt. Von 43 dieser 67 Blutproben standen tumorzellfreie Leukozytenfraktionen zur Verfügung, die in derselben Weise wie die tumorzellangereicherten Proben behandelt wurden. Die amplifizierten cDNAs wurden einem RT-PCR-Verfahren unterworfen. Alle Proben wurden doppelt analysiert. Insgesamt wurden 20 Gene einschließlich des Housekeeping Gens B2M analysiert. Drei Proben, die keine B2M Expression zeigten, wurden von der weiteren Analyse ausgeschlossen. Es standen daher 23 tumorzellangereicherte Proben und weitere 41 tumorzellangereicherte Proben sowie entsprechende tumorzellfreie Proben zur Verfügung. Dreiundzwanzig tumorzellangereicherte Proben zeigten Genexpression für zumindest ein Markergen. Unter diesen zeigten die entsprechenden 40 tumorzellfreien Blutproben keine Expression für die untersuchten Gehe. Nur eine Probe war sowohl in der tumorzellangereicherten als auch ln der tumorzellfreien Blutprobe für TM4SF1 positiv. FN1 war bemerkenswerter Weise in 14 tumorzellgereicherten Proben exprimiert. Mit einer Kombination der Marker FN1, TM4FS1 und RBPMS werden 19 der insgesamt 23 (82,6%) positiven Blutproben gefunden, während zwei der verbleibenden exklusiv NTS und eine Probe exklusiv GHR exprimierten. Interessanter Weise wurden bis auf eine der tumorzellabgereicherten Fraktionen (erhalten bei der Dichtegradientenzentrifugation) keine Expression der Markergene gefunden, außer für das Kontroligen B2M. Die Ergebnisse für Proben die eine Expression von Markergenen zeigten sind in der folgenden Tabelle zusammengefasst.

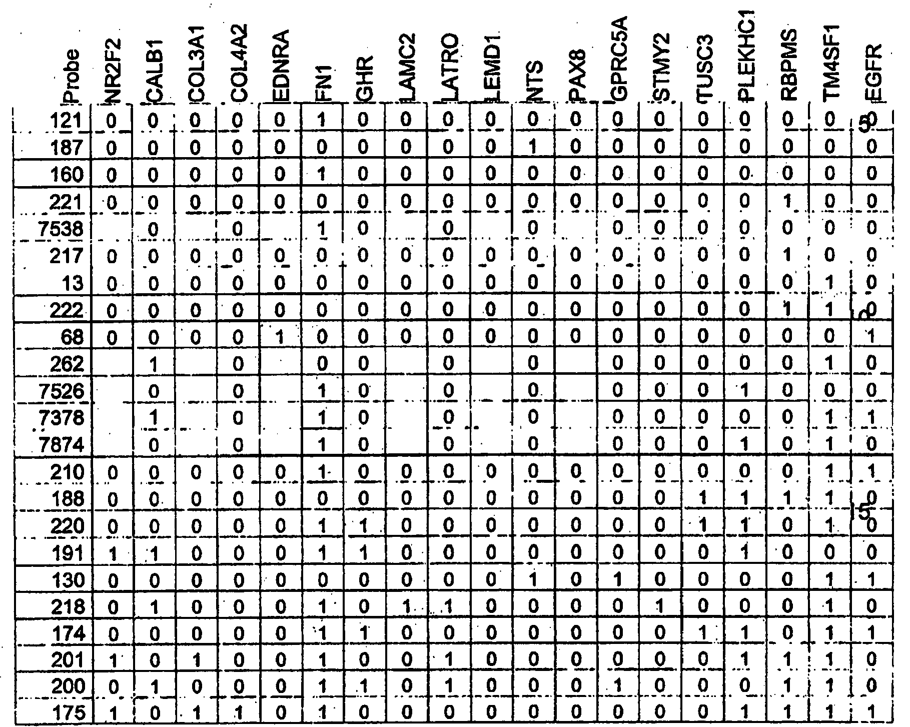

Weiters wurde in 16 Kontrollen keine Expression der Markergene gefunden. Es muss erwähnt werden, dass nur jeweils eine einzige Kontrollprobe ausschließlich für die Markergene FN1, ENDRA oder RBPMS positiv war. Zwei Kontrollproben waren für die Markerkombination TM4SF1 und PLEKHC1 positiv. Da keine entsprechende tumorzellenfreie Fraktion zu dieser Kontrollprobe vorhanden war, ist eine abschließende Interpretation dieses Ergebnisses nicht möglich.

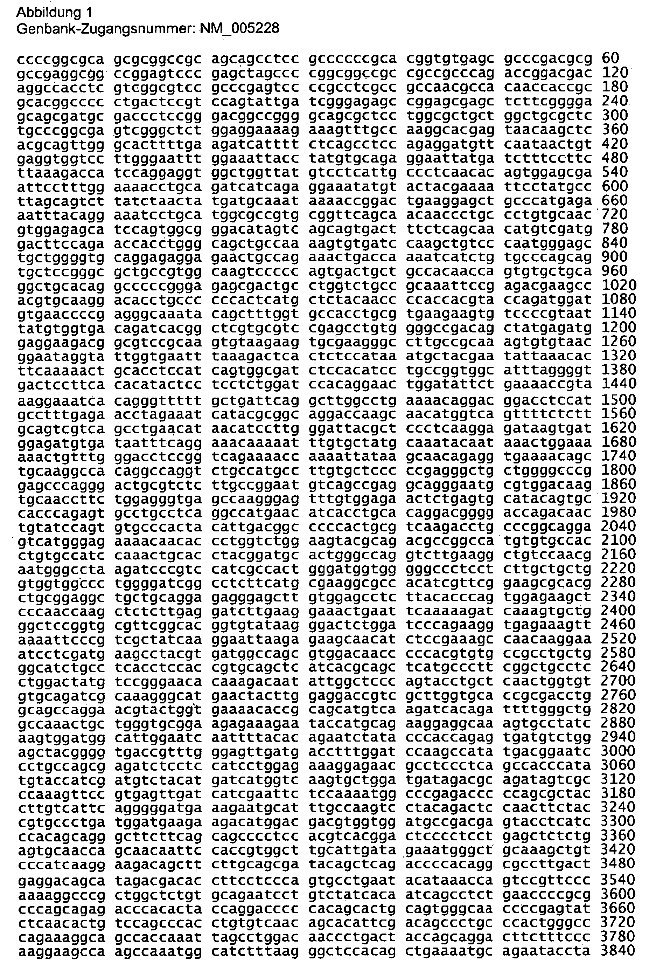

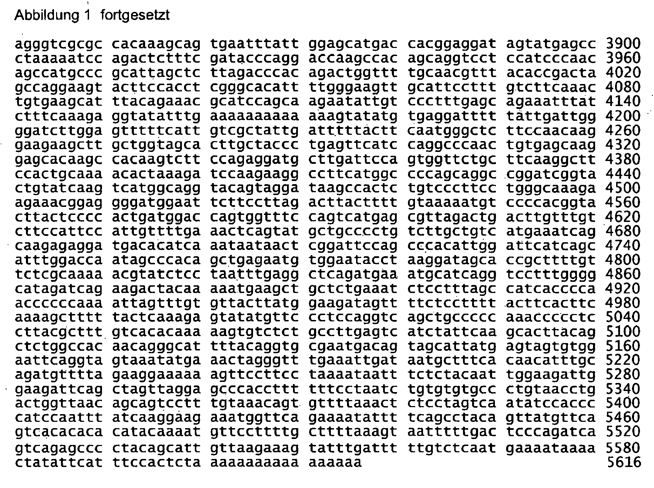

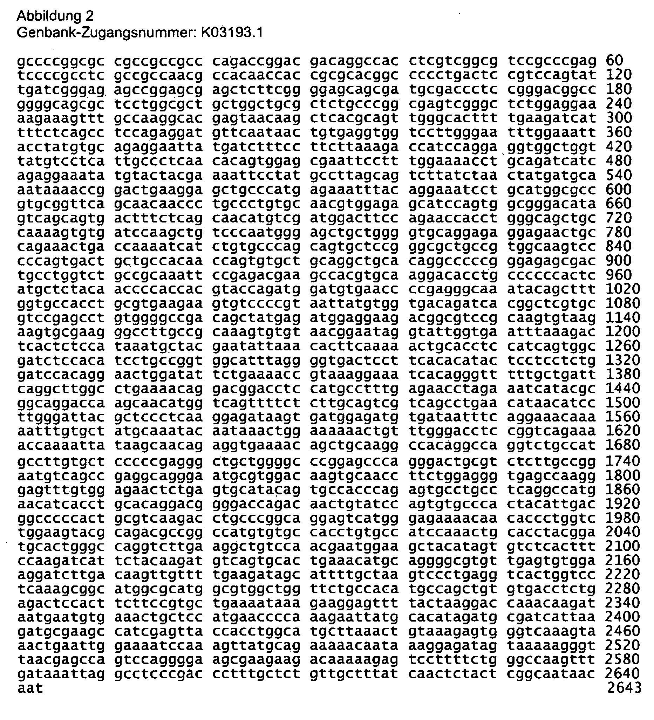

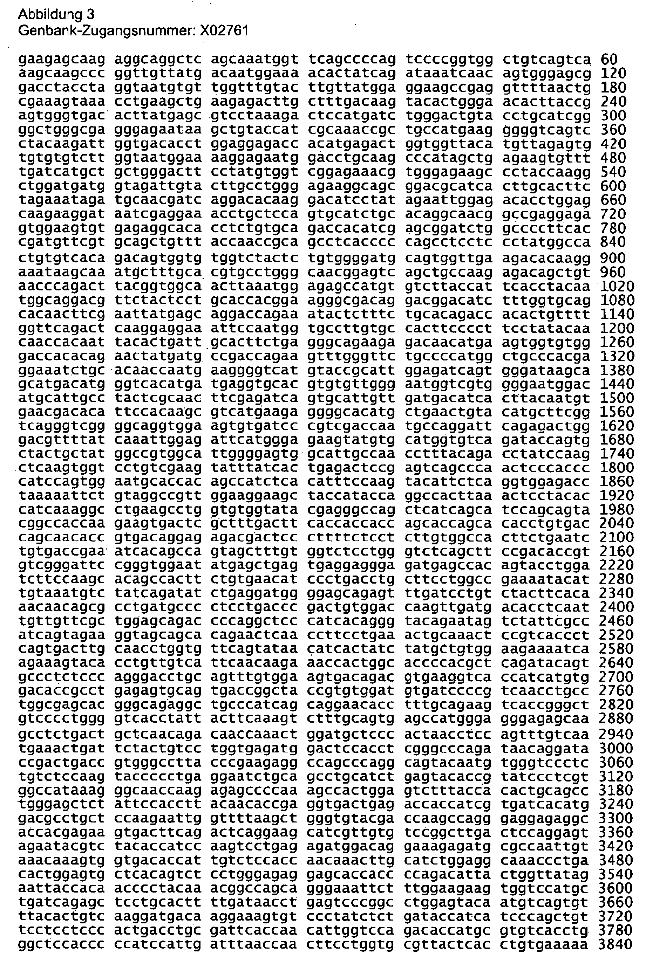

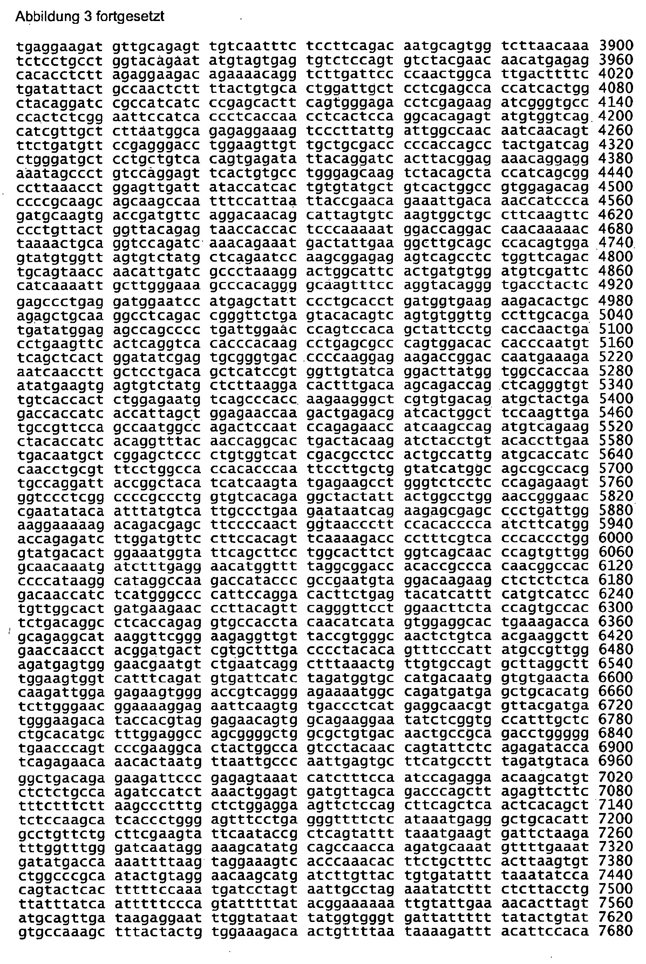

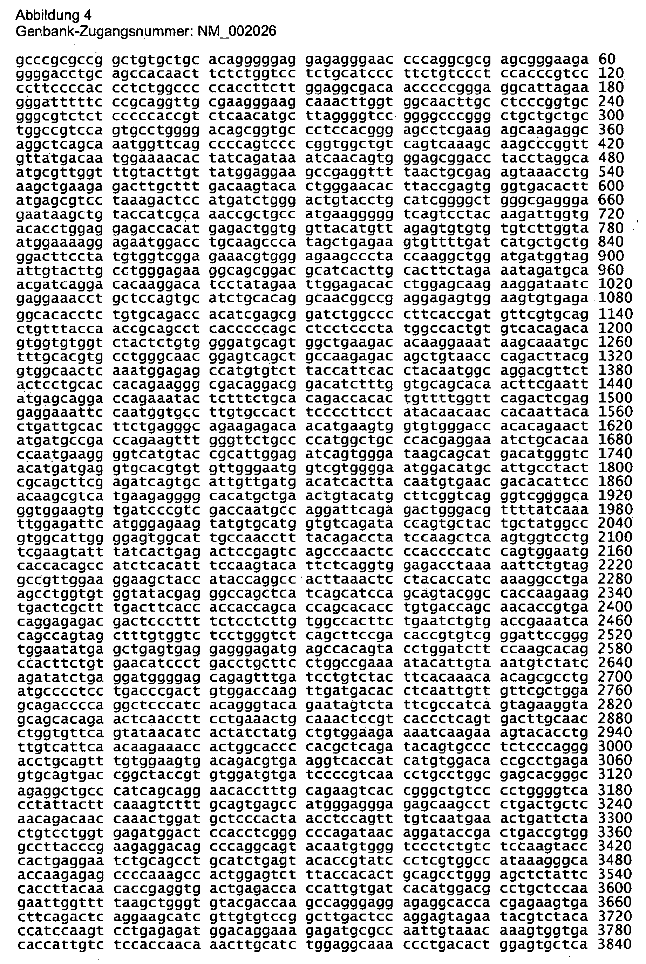

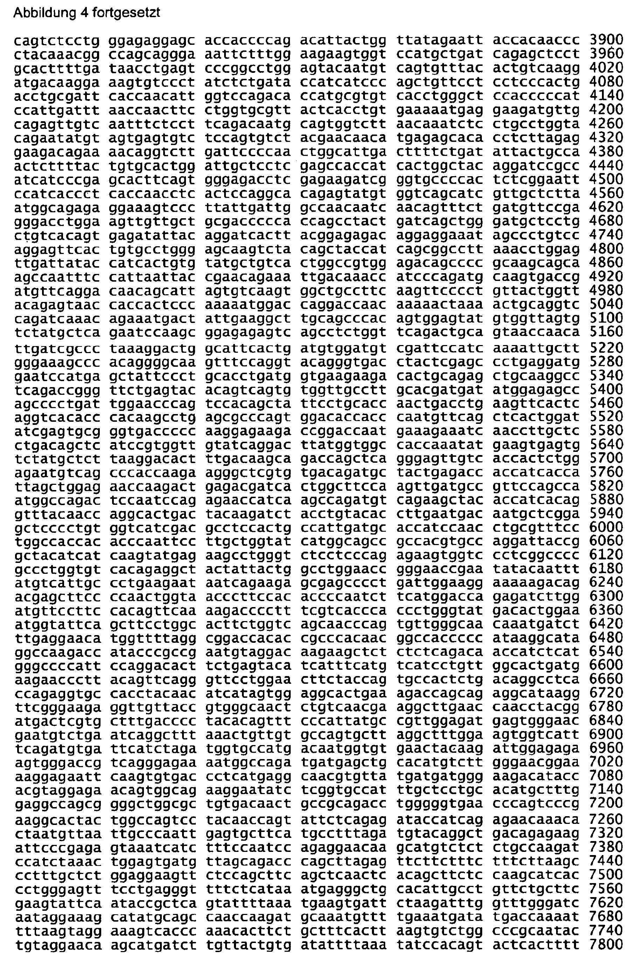

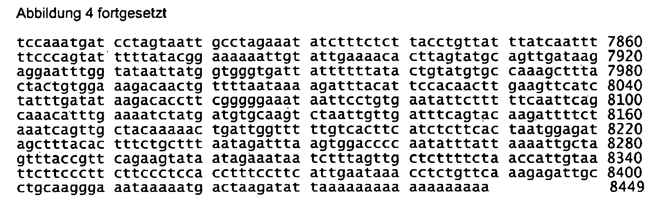

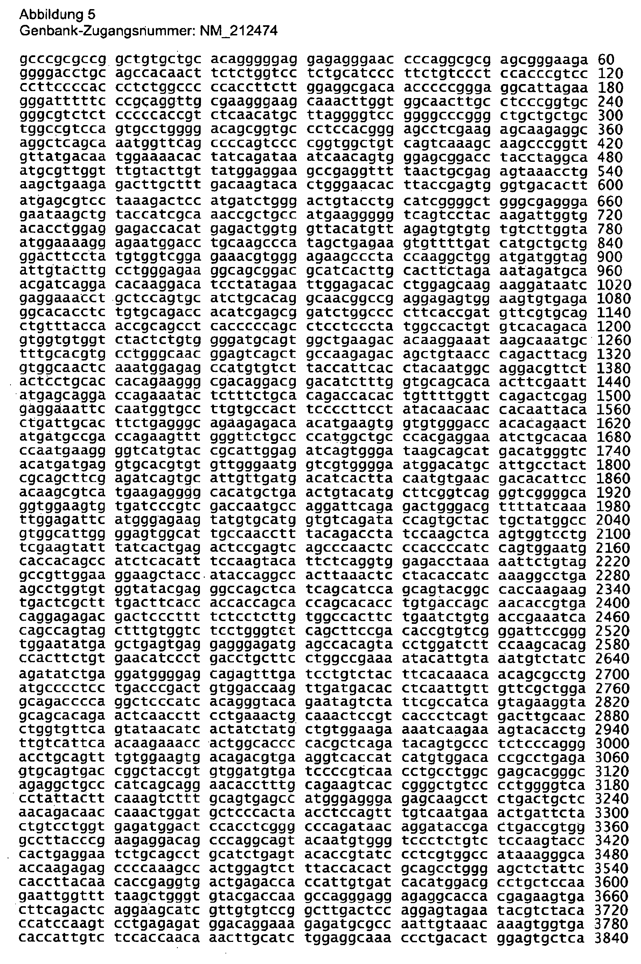

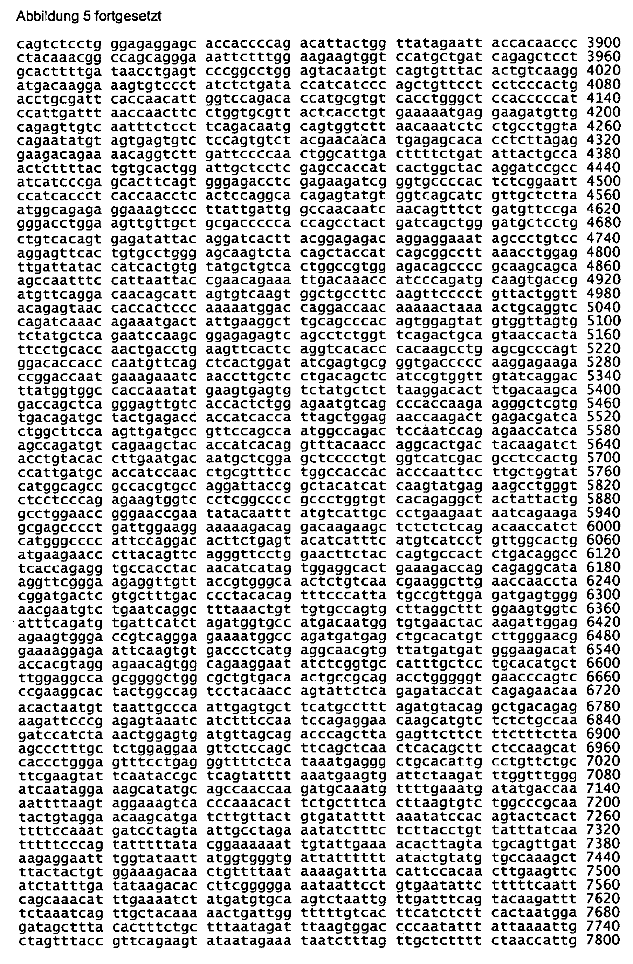

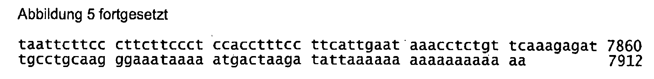

## Patentansprüche

1. Verfahren zur Diagnose von Eierstockkrebs als Primärtumor durch den Nachweis von disseminierten Tumorzellen in Blut von Eierstockkrebspatientinnen, indem untersucht wird, ob eine Genexpression für ein oder mehrere bestimmte Gene stattfindet, **dadurch gekennzeichnet, dass** in der Blutprobe Tumorzellen angereichert werden und die Gene ausgewählt aus der Gruppe bestehend aus NR2F2, LEMD1, CALB1, LAMC2, FN1, STMY2, COL4A2, GPRC5A, NTS, GHR, EDNRA, TUSC3, PLEKHC1, RBPMS, die EGFR Variante mit der Genbank-Zugangsnummer NM_005228 und die EGFR Variante mit der Genbank-Zugangsnummer K03193.1 sind.

2. Verfahren nach Anspruch 1, **dadurch gekennzeichnet, dass** als Gen für FN1 die Varianten fibronectin1 mit der GenBank-Zugangsnummer X02761, fibronectin1 isoform3 mit der GenBank-Zugangsnummer NM_002026, und/oder fibronectin1 isoform6 mit der GenBank-Zugangsnummer NM_212474 ausgewählt werden.

3. Verfahren nach Anspruch 1 oder 2, wobei mehrere der Gene ausgewählt werden.

4. Verfahren nach einem der Ansprüche 1 bis 3, wobei die Genexpression mit einem RNA basierenden Test bestimmt wird.

## Claims

1. A method for diagnosing ovarian cancer as a primary tumour by detecting disseminated tumour cells in blood from ovarian cancer patients, in which an examination is carried out as to whether a gene expression for one or more specific genes occurs, **characterized in that** tumour cells are enriched in the blood sample and the genes are selected from the group consisting of NR2F2, LEMD1, CALB1, LAMC2, FN1, STMY2, COL4A2, GPRC5A, NTS, GHR, EDNRA, TUSC3, PLEKHC1, RBPMS, the EGFR variant with the gene bank accession number NM_005228 and the EGFR variant with the gene bank accession number K03193.1.

2. The method as claimed in claim 1, **characterized in that** the gene for FN1 is selected from the variants fibronectin1 with the gene bank accession number X02761, fibronectin1 isoform3 with the gene bank accession number NM_002026 and/or fibronectin1 isoform6 with the gene bank accession number NM_212474.

3. The method as claimed in claim 1 or claim 2, wherein several of the genes are selected.

4. The method as claimed in one of claims 1 to 3, wherein the gene expression is determined using an RNA-based test.

## Revendications

1. Procédé de diagnostic du cancer de l'ovaire en tant que tumeur primaire par la mise en évidence de cellules tumorales disséminées dans le sang des patientes atteintes du cancer de l'ovaire en analysant si une expression génique d'un ou plusieurs gènes définis a lieu, **caractérisé en ce que** les cellules tumorales sont enrichies dans le prélèvement sanguin et que les gènes sont sélectionnés dans le groupe composé de NR2FE, LEMD1, CALB1, LAMC2, FN1, STMY2, COL4A2, GPRC5A, NTS, GHR, EDNRA, TUSC3, PLEKHC1, RBPMS, la variante EGFR portant le numéro d'accès à la banque génétique NM_005228 et la variante EGFR portant le numéro d'accès à la banque génétique K03193.1.

2. Procédé selon la revendication 1, **caractérisé en ce qu'**on sélectionne comme gène pour FN1 les variantes fibronectine 1 portant le numéro d'accès à la banque génétique X02761, fibronectine 1 isoform3 portant le numéro d'accès à la banque génétique NM_002026 et/ou fibronectine 1 isoform6 portant le numéro d'accès à la banque génétique NM_212474.

3. Procédé selon la revendication 1 ou 2, dans lequel plusieurs des gènes sont sélectionnés.

4. Procédé selon une des revendications 1 à 3, dans lequel l'expression génique est déterminée par un test basé sur l'ARN.
